(19) Europäisches Patentamt
European Patent Office
Office européen des brevets

(11) EP 2 015 726 B1

(12) # EUROPÄISCHE PATENTSCHRIFT

(45) Veröffentlichungstag und Bekanntmachung des Hinweises auf die Patenterteilung:
**29.12.2010 Patentblatt 2010/52**

(21) Anmeldenummer: **06742745.0**

(22) Anmeldetag: **28.04.2006**

(51) Int Cl.:
***A61K 8/64*** (2006.01) ***A61Q 19/08*** (2006.01)
***C07K 5/00*** (2006.01) ***C07K 5/10*** (2006.01)

(86) Internationale Anmeldenummer:
**PCT/EP2006/003998**

(87) Internationale Veröffentlichungsnummer:
**WO 2007/124770 (08.11.2007 Gazette 2007/45)**

(54) **KOSMETISCHE ZUSAMMENSETZUNG ZUR STIMULIERUNG DER SYNTHESE DER PROTEINE DER BASALMEMBRAN**

COSMETIC COMPOSITION FOR STIMULATING THE SYNTHESIS OF PROTEINS OF THE BASEMENT MEMBRANE

COMPOSITION COSMÉTIQUE POUR STIMULER LA SYNTHÈSE DES PROTÉINES DE LA MEMBRANE BASALE

(84) Benannte Vertragsstaaten:
**AT BE BG CH CY CZ DE DK EE ES FI FR GB GR HU IE IS IT LI LT LU LV MC NL PL PT RO SE SI SK TR**

(43) Veröffentlichungstag der Anmeldung:
**21.01.2009 Patentblatt 2009/04**

(73) Patentinhaber: **DSM IP Assets B.V.**
**6411 TE Heerlen (NL)**

(72) Erfinder:
- **HEIDL, Marc**
  **79639 Grenzach-Wyhlen (DE)**
- **STÖCKLI, Martin**
  **CH-7117 Burg im Leimental (CH)**
- **IMFELD, Dominik**
  **CH-4142 Münchenstein (CH)**
- **ZIEGLER, Hugo**
  **CH-4108 Witterswil (CH)**

(74) Vertreter: **Braun, André jr.**
**Braunpat Braun Eder AG**
**Reussstrasse 22**
**Postfach**
**4015 Basel (CH)**

(56) Entgegenhaltungen:
**EP-A- 1 640 041 WO-A-2004/099237**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 015 726 B1

SEQUENCE LISTING

<110> DSM IP Assets B.V.
<120> Cosmetic Composition for stimulating the synthesis of Proteins of the Basement Membrane
<130> P15369EP00
<140> EP 06 742 745.0 - 1521
<141> 2008-09-24
<150> PCT/EP2006/003998
<151> 2006-04-28
<160> 13
<170> PatentIn version 3.5
<210> 1
<211> 4
<212> PRT
<213> synthetic tetrapeptide derivative
<220>
<221> MOD_RES
<222> (1)..(1)
<223> Palm-Lys
<400> 1

```
Lys Val Lys Ala
1
```

<210> 2
<211> 4
<212> PRT
<213> synthetic tetrapeptide derivative
<220>
<221> MOD_RES
<222> (1)..(1)

<223> Palm-Lys
<400> 2

Lys Val Lys Arg
1

<210> 3
<211> 4
<212> PRT
<213> synthetic tetrapeptide derivative
<220>
<221> MOD_RES
<222> (1)..(1)
<223> Palm-Lys
<400> 3

Lys Val Lys Gln
1

<210> 4
<211> 4
<212> PRT
<213> synthetic tetrapeptide derivative
<220>
<221> MOD_RES
<222> (1)..(1)
<223> Palm-Lys
<400> 4

Lys Val Lys Ser
1

<210> 5
<211> 4
<212> PRT
<213> synthetic tetrapeptide derivative
<220>
<221> MOD_RES
<222> (1)..(1)
<223> Palm-Lys
<220>
<221> MISC_FEATURE
<222> (3)..(3)
<223> Xaa is Dab
<400> 5

Lys Val Xaa Glu
1

<210> 6
<211> 4
<212> PRT
<213> synthetic tetrapeptide derivative

<220>
<221> MOD_RES
<222> (1)..(1)
<223> Palm-Lys
<220>
<221> MISC_FEATURE
<222> (3)..(3)
<223> Xaa is Dab
<400> 6

```
Lys Val Xaa Asp
1
```

<210> 7
<211> 4
<212> PRT
<213> synthetic tetrapeptide derivative
<220>
<221> MOD_RES
<222> (1)..(1)
<223> Palm-Lys
<220>
<221> MISC_FEATURE
<222> (3)..(3)
<223> Xaa is Dab
<400> 7

```
Lys Val Xaa Thr
1
```

<210> 8
<211> 4
<212> PRT
<213> synthetic tetrapeptide derivative
<220>
<221> MOD_RES
<222> (1)..(1)
<223> Palm-Lys
<220>
<221> MISC_FEATURE
<222> (3)..(3)
<223> Xaa is Dab
<400> 8

```
Lys Val Xaa Lys
1
```

<210> 9
<211> 4
<212> PRT
<213> synthetic tertapeptide derivative
<220>
<221> MOD_RES
<222> (1)..(1)
<223> Palm-Lys

<220>
<221> MISC_FEATURE
<222> (3)..(3)
<223> Xaa is Dab
<400> 9

Lys Val Xaa Met
1

<210> 10
<211> 4
<212> PRT
<213> syntehtic tetrapeptide derivative
<220>
<221> MOD_RES
<222> (1)..(1)
<223> Palm-Lys
<220>
<221> MISC_FEATURE
<222> (3)..(3)
<223> Xaa is Dab
<400> 10

Lys Val Xaa Asn
1

<210> 11
<211> 4
<212> PRT
<213> synthetic tetrapeptide derivative
<220>
<221> MOD_RES
<222> (1)..(1)
<223> Palm-Lys
<220>
<221> MISC_FEATURE
<222> (3)..(3)
<223> Xaa is Dab
<400> 11

Lys Val Xaa His
1

<210> 12
<211> 4
<212> PRT
<213> synthetic tetrapeptide derivative
<220>
<221> MOD_RES

<222> (1)..(1)
<223> Palm-Lys

<220>
<221> MISC_FEATURE
<222> (3)..(4)
<223> Xaa in pos. 3 is Dab and in pos. 4 is Nle
<400> 12

```
Lys Val Xaa Xaa
1
```

<210> 13
<211> 4
<212> PRT
<213> synthetic tetrapeptide derivative
<220>
<221> MOD_RES
<222> (1)..(1)
<223> Palm-Lys
<220>
<221> MISC_FEATURE
<222> (3)..(3)
<223> Xaa is Dab
<400> 13

```
Lys Val Xaa Phe
1
```

## Beschreibung

Die vorliegende Erfindung betrifft eine kosmetische Zusammensetzung, insbesondere eine topisch applizierbare kosmetischen Zusammensetzung, enthaltend mindestens zwei bestimmte Peptid-Derivate, zur Stimulierung der Synthese der Moleküle der Basalmembran, insbesondere deren Proteine, sowie die Verwendung dieser bestimmten Peptid-Derivate zur Stimulierung der Synthese der Moleküle der Basalmembran, insbesondere deren Proteine.

Die Basalmembran (BM) an der dermal-epidermalen Verbindung hat mehrere Funktionen, wovon die offensichtlichste Funktion die enge Verbindung der Epidermis mit der Dermis ist und so den mechanisch stabilen Zusammenhalt der zwei Gewebeschichten gewährleistet. Auch die "Polarität" und der Aufbau der Epidermis wird durch die Basalmembran beeinflusst und gleichzeitig ist die BM eine klare Abgrenzung zwischen Epidermis und Dermis. Man geht davon aus, dass die BM die epidermale Differenzierung der Keratinozyten einleitet und den proliferativen Zustand der basalen Zellschicht aufrechterhält. Unter normalen Bedingungen verhindert die BM auch den direkten Kontakt von epidermalen Zellen mit der Dermis. Nach einer Verletzung mit Schädigung der BM kommt es jedoch zum direkten Kontakt mit der Dermis worauf die Zellen ihr Verhalten ändern und den Wundheilungsprozess einleiten.

Eine weitere wichtige Funktion der BM beinhaltet die korrekte Kommunikation zwischen den epidermalen und den dermalen Zellen. Da Epidermis und die Dermis nicht unabhängig voneinander funktionieren, benötigt die normale Hauthomeostase die regelmässige Passage von biochemischen Signalen in beiden Richtungen zwischen den zwei Zelltypen. Im Allgemeinen sind das kleine Moleküle die in einem Kompartiment hergestellt werden und selektiv über die BM transportiert werden müssen, um ihre "Botschaft" auf der anderen Seite zu vermitteln. Der BM kommt dabei die wichtige Funktion zu, als aktiver Filter zu wirken und die Signalmoleküle bedarfsgerecht weiterzuleiten oder eben zu blockieren. Die richtig funktionierende epidermal-dermale Kommunikation über die BM ist essentiell für die Haut.

Die BM selber kann wiederum morphologisch in drei Schichten unterteilt werden, die Lamina lucida, die Lamina densa und die Lamina fibroreticularis. Die Lamina lucida ist die Region zwischen den epidermalen Zellen und der Lamina densa und enthält die Hemidesmosemen, welche im EM als elektronendichte Plaques sichtbar sind. Die Hemidesmosomen verbinden die basalen, proliferationsfähigen Keratinozyten mit der BM und sind unter anderem aus dem Kollagen XVII (oder bp180) und den Integrinen alpha6/beta4 aufgebaut. Deletionen im Kollagen XVII können zu fragiler Haut mit häufiger Blasenbildung führen (Epidermolysis Bullosa Simplex). Die Lamina densa ist eine flache Struktur (Sheet) hauptsächlich bestehend aus Kollagen IV.

Bestandteile der BM sind im Wesentlichen Proteine, Proteoglykane und Glykosaminoglykane. Einer der wichtigen Komponenten des Verankerungskomplexes ist Laminin V. Laminin V ist unter anderem essentiell für die epidermale Anheftung an das dermale Gewebe, da Mutationen in Laminin V ebenfalls zu schweren Formen von Blasenbildung der Haut führen (Herlitz'junctional epidermolysis bullosa). Laminin V bindet die transmembranären Integrine alpha6/beta4 der Hemidesmosomen einerseits, auf der anderen Seite ist Laminin V mit Kollagen VII verbunden, das wiederum Verankerungsfibrillen in die Dermis bildet. Das Kollagen VII ist die Hauptkomponente der Lamina fibroreticularis. Die Strukturproteine Kollagen IV, VII, XVII sowie Laminin V und die Integrine alpha6, beta4 sind demnach neben weiteren Proteinen essentielle Hauptkomponenten für den Aufbau der BM sowie der Hemidesmosomen und damit wichtig für die korrekten und vielseitigen Funktionen der BM in der Haut.

Es ist bekannt, dass sich die endogene (altersbedingte) oder exogene (lichtbedingte) Alterung in einer irreversiblen Degenerierung der Gewebe, insbesondere der Haut manifestiert und makroskopisch in Form von Falten, Schlaffheit, rauer Oberfläche, unregelmässiger Pigmentierung etc. zunehmend sichtbar wird. Diese Änderungen entstehen durch eine Reduktion der anabolischen Reaktionen (Synthesen) und eine Erhöhung der katabolischen Reaktionen (Abbau) von Kollagenen unter anderem auch den Proteinkomponenten der BM. Histologsch sichtbare Veränderungen sind die Anreicherung von verkürzten unstrukturierten Elastinfasern (Elastosis), einer Reduktion der Kollagenfasern, der Infiltration von Entzündungsmediatoren, einer Atropie der Epidermis mit atypischen, unpolaren Keratinozyten. Zum Beispiel nehmen die Anzahl der Verankerungsfibrillen von der BM in die Dermis und die Menge an Kollagen VII, aus dem die Fibrillen bestehen, in lichtgealterter Haut schnell ab und damit wird die Stabilität der Bindung der BM an die Dermis gestört. Auch die anderen Proteine der BM werden teilweise in alternder Haut zurückgebildet und die strukturelle Organisation der BM degeneriert mit zunehmendem Alter.

Die erwähnten Strukturproteine der BM werden vorwiegend durch Keratinozyten exprimiert und teilweise auch durch Fibroblasten. Die Synthese-Reaktionen in der Hautmatrix werden hauptsächlich durch Polypeptide, den sog. Wachstumsfaktoren und Zytokinen, reguliert. Unter diesen Peptiden ist TGF-$\beta$1 einer der wichtigsten, in die Synthesereaktionen dieser Hautmatrix involvierten Regulatoren. Er wird in der Matrix auch durch Keratinozyten und Fibroblasten sezerniert. Extern zugefügte Wirkstoffe, welche die Synthese der BM Proteine anregen, könnten einerseits altersbedingte Degenerationen der BM kompensieren und auch schon präventiven Charakter haben, indem die mit dem Altern verbundene Abnahme des BM Proteingehaltes gebremst wird.

WO 2004/099237 offenbart Tripeptide für die kosmetische Verwendung zur Behandlung der Hautalterung.

Es wurde nun gefunden, dass es überraschenderweise gelingt, mit einer Kombination von mindestens einem bestimmten kosmetisch wirksamen Tri- und mindestens einem bestimmten kosmetisch wirksamen Tetrapeptid-Derivat

(im weiteren "erfindungsgemässe Verbindungen" genannt), welche in topisch applizierbaren, kosmetischen Zusammensetzungen vorliegen, eine deutliche Verbesserung des Hautbildes und eine Verlangsamung des altersbedingten Abbaus der Basalmembranproteine zu erreichen. Das geschieht dadurch, dass die erfindungsgemässen Verbindungen schnell und in ausreichender Konzentration durch das Stratum Corneum und die Epidermis zum Wirkort im Grenzbereich zwischen Epidermis und Dermis diffundieren können und dort eine schnelle und starke Stimulierung der Synthese der Proteine der Basalmembran bewerkstelligen. So kann gezeigt werden, dass mit einer Kombination von mindestens einem bestimmten Tripeptid-Derivat und mindestens einem bestimmten Tetrapeptid-Derivat die Konzentration von Kollagen IV, VII, XVII, Laminin V und jene des Integrins beta4 signifikant erhöht wird.

Die vorliegende Erfindung ist in den Patentansprüchen definiert. Insbesondere betrifft die vorliegende Erfindung eine kosmetische Zusammensetzung, insbesondere eine topisch applizierbare kosmetische Zusammensetzung, enthaltend mindestens eine Verbindung der allgemeinen Formel (I),

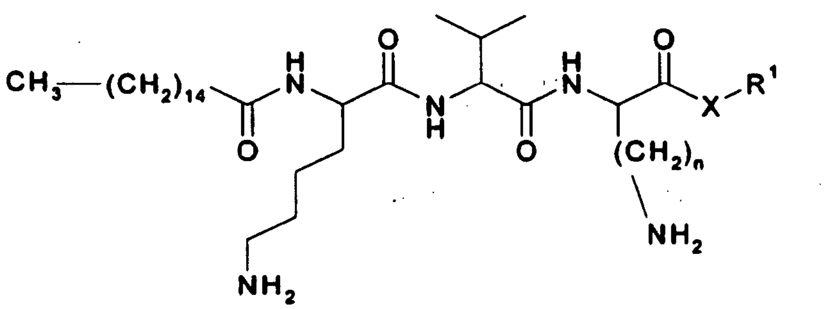

(I)

worin

$R^1$ H, $C_1$-$C_{20}$-Alkyl, Cycloalkyl oder Aryl-$C_1$-$C_4$-Alkyl,
n 1-4,
X -O-,-NH- oder -$NR^2$- und
$R^2$ H oder $C_1$-$C_{20}$-Alkyl bedeuten;

sowie mindestens eine Verbindung entsprechend der obigen Formel (I), worin aber
$XR^1$ mit X in der Bedeutungsmöglichkeit -NH- den Rest einer alpha-Aminosäure bedeutet.

Die vorliegende Erfindung betrifft auch die Verwendung der Verbindungen der obigen Formel (I) zusammen mit Verbindungen entsprechend der obigen Formel (I), worin aber $XR^1$ mit X in der Bedeutungsmöglichkeit -NH- den Rest einer alpha-Aminosäure bedeutet, zur Herstellung der vorgehend definierten Zusammensetzung und deren Verwendung zur Stimulierung der Synthese der Moleküle der Basalmembran, insbesondere deren Proteine.

Die vorliegende Erfindung betrifft auch die Verwendung der Verbindungen der obigen Formel (I) zusammen mit Verbindungen entsprechend der obigen Formel (I), worin aber $XR^1$ mit X in der Bedeutungsmöglichkeit -NH- den Rest einer alpha-Aminosäure bedeutet, zur Stimulierung der Synthese der Moleküle der Basalmembran, insbesondere deren Proteine.

Die vorliegende Erfindung betrifft auch diejenigen Verbindungen der obigen allgemeinen Formel (I), worin X -$NR^2$- bedeutet und sowohl $R^1$ als auch $R^2$ von H verschiedene sind, als auch die Verbindungen entsprechend der obigen allgemeinen Formel (I), worin aber $XR^1$ mit X in der Bedeutungsmöglichkeit -NH- den Rest einer alpha-Aminosäure bedeutet, als solche.

In den Verbindungen der Formel (I) und in den Verbindungen entsprechend der obigen allgemeinen Formel (I), worin aber $XR^1$ mit X in der Bedeutungsmöglichkeit -NH- den Rest einer alpha-Aminosäure bedeutet, können die Aminosäuren als Racemate oder in ihrer enantiomerenreinen L und D Form vorliegen.

Die oben verwendeten allgemeinen Ausdrücke sind wie folgt definiert:

Unter "Alkyl" sind sowohl lineare als auch verzweigte gesättigte Kohlenwasserstoffreste zu verstehen. Beispiele sind Methyl, Ethyl, Propyl, n-Butyl, n-Pentyl, n-Hexyl, n-Heptyl, n-Octyl, n-Nonyl, n-Undecanyl, n-Dodecanyl, n-Tridecanyl, n-Hexadecanyl, n-Heptadecanyl, n-Octadecanyl oder n-Nonadecanyl als unverzweigte und Isopropyl, tert.Butyl, Isobutyl, sec.-Butyl, Isoamyl als verzweigte Reste.

Unter "Cycloalkyl" sind zyklische gesättigte Kohlenwasserstoffreste mit bis zu 8 Kohlenstoffatomen zu verstehen, wie etwa Cylopropyl, Cyclobutyl, Cyclopentyl, Cyclohexyl oder Cycloheptyl.

Unter "Aryl" sind aromatische, ein- oder mehrkernige aromatische Kohlenwasserstoffreste mit bis zu 10 Kohlenstoffatomen zu verstehen, welche durch beispielsweise Alkyl, Alkoxy, Halogen und/oder Trifluormethyl ein- oder mehrfach substituiert sein können, wie etwa Phenyl, p- Tolyl, o-Tolyl-, m-Tolyl, 3,4-Dimethoxyphenyl, 2-Naphthyl oder 3-Naphthyl. Weiterhin umfasst "Aryl" auch Reste von Heteroarylgruppen, d.h. von gegebenenfalls entsprechend substituierten, ein- oder mehrkernigen aromatischen heterocyclischen Resten, wie etwa 2-Pyridyl, 3-Pyridyl, 4-Pyridyl, 2-Chinolinyl oder 3-Isochinolinyl.

Die erfindungsgemässen Verbindungen können mit Säuren einoder mehrzählige, einheitliche oder gemischte Salze bilden, z.B. mit anorganischen Säuren, wie Chlorwasserstoff, Bromwasserstoff, Schwefelsäure oder Phosphorsäure; oder mit geeigneten Carbonsäuren, z.B. aliphatischen Mono- oder Dicarbonsäuren, wie Ameisensäure, Essigsäure, Trifluoressigsäure, Trichloressigsäure, Propionsäure, Glykolsäure, Bernsteinsäure, Fumarsäure, Malonsäure, Maleinsäure, Oxalsäure, Phthalsäure, Zitronensäure, Milchsäure oder Weinsäure; oder mit aromatischen Carbonsäuren, wie Benzoesäure oder Salicylsäure; oder mit aromatisch-aliphatischen Carbonsäuren, wie Mandelsäure oder Zimtsäure; oder mit heteroaromatischen Carbonsäuren, wie Nikotinsäure; oder mit aliphatischen oder aromatischen Sulfonsäuren; wie Methansulfonsäure oder Toluolsulfonsäure. Bevorzugt sind dermatologisch verträgliche Salze.

Die allgemeine Formel (I) umfasst alle isomeren Formen, sowie deren Gemische, z.B. racemische Gemische und Gemische von Rotameren.

Bevorzugt sind Kombinationen von Verbindungen der allgemeinen Formel (I), worin $R^1$ H oder $C_1$-$C_{20}$-Alkyl, n 2 oder 4 und X Sauerstoff bedeuten, mit Verbindungen entsprechend der allgemeinen Formel (I), worin aber $XR^1$ mit X in der Bedeutungsmöglichkeit -NH- den Rest einer natürlichen alpha-Aminosäure bedeutet und n 2 bedeutet.

Besonders bevorzugt sind die Kombinationen von Verbindungen der folgenden Tabelle 1:

1.1 Palm-Lys-Val-Lys-OH
1.2 Palm-Lys-Val-Orn-OH
1.3 Palm-Lys-Val-Dab-OH
1.4 Palm-Lys-Val-Dab-OMe
1.5 Palm-Lys-Val-Dab-OOctyl
1.6 Palm-Lys-Val-Dab-OCetyl
1.7 Palm-Lys-Val-Dab-$NH_2$
1.8 Palm-Lys-Val-Dab-NHButyl
1.9 Palm-Lys-Val-Dab-$N(Butyl)_2$
1.10 Palm-Lys-Val-Dab-NHOctyl
1.11 Palm-Lys-Val-Dab-$N(Octyl)_2$
1.12 Palm-Lys-Val-Dab-NHCetyl
1.13 Palm-Lys-Val-Dab-$N(Cetyl)_2$ oder
1.14 Palm-Lys-Val-Dap-OH

mit

2.1 Palm-Lys-Val-Lys-Ala-OH
2.2 Palm-Lys-Val-Lys-Arg-OH
2.3 Palm-Lys-Val-Lys-Gln-OH
2.4 Palm-Lys-Val-Lys-Ser-OH
2.5 Palm-Lys-Val-Dab-Glu-OH
2.6 Palm-Lys-Val-Dab-Asp-OH
2.7 Palm-Lys-Val-Dab-Thr-OH
2.8 Palm-Lys-Val-Dab-Lys-OH
2.9 Palm-Lys-Val-Dab-Met-OH
2.10 Palm-Lys-Val-Dab-Asn-OH
2.11 Palm-Lys-Val-Dab-His-OH
2.12 Palm-Lys-Val-Dab-Nle-OH oder
2.13 Palm-Lys-Val-Dab-Phe-OH

Ganz besonders bevorzugte Kombinationspartner sind Palm-Lys-Val-Dab-OH (1.3)und Palm-Lys-Val-Dab-Thr-OH (2.7).

Die erfindungsgemässen Verbindungen können in Konzentrationen verwendet werden, die zwischen 0,5 und 5000 ppm (w/w), vorzugsweise zwischen 1 und 1000 ppm (w/w), im kosmetischen Endprodukt variieren.

Die erfindungsgemässen Verbindungen können in Form einer Lösung, einer Dispersion, einer Emulsion oder eingekapselt in Träger wie die Makro-, Mikro- oder Nanokapseln, in Liposomen oder Chylomikronen, oder eingeschlossen

in Makro-, Mikro- oder Nanoteilchen oder in Mikroschwämme oder absorbiert auf pulverförmigen organischen Polymeren, Talk, Bentonit und anderen mineralischen Trägern verwendet werden.

Die erfindungsgemässen Verbindungen können in jeder galenischen Form verwendet werden: Emulsionen Öl/Wasser und Wasser/Ö1, Milch, Lotionen, Salben, gelierende und viskose, spannungsaktive und emulgierende Polymere, Pommaden, Shampoos, Seifen, Gele, Puder, Sticks und Stifte, Sprays, Körperöle, Gesichtsmasken, Pflaster.

Die erfindungsgemässen Verbindungen können mit jedem anderen üblicherweise verwendeten Inhaltsstoff angewendet werden. Solche Zusammensetzungen können Extraktionslipide und/oder Syntheselipide, gelierende und viskose, spannungsaktive und emulgierende Polymere, wasser- oder fettlösliche Wirkprinzipien, Pflanzenextrakte, Gewebeextrakte, Meeresextrakte, Sonnenschutzmittel, Antioxidantien, Feuchthalteund Barrieremittel, Haut revitalisierende Wirkstoffe, zusätzliche Hautpflegewirkstoffe oder Hautschutzmittel enthalten.

Die erfindungsgemässen Verbindungen können mit jedem anderen üblicherweise verwendeten kosmetischen Hautpflegewirkstoff kombiniert verwendet werden. Beispielhaft für einen zusätzlichen Hautpflegewirkstoff seien Anti-Faltenwirkstoffe/Anti-Atrophiewirkstoffe genannt: Die erfindungsgemässen Zusammensetzung können eine sichere und wirksame Menge von einem oder mehreren Anti-Faltenwirkstoffen oder Anti-Atrophiewirkstoffen enthalten. Beispielhafte Anti-Falten/Anti-Atrophie-Wirkstoffe, die zur Verwendung in den erfindungsgemässen Zusammensetzungen geeignet sind, schliessen schwefelhaltige D- und L-Aminosäuren und ihre Derivate und Salze, insbesondere die N-Acetylderivate, wobei ein bevorzugtes Beispiel hierfür N-Acetyl-L-cystein ist; Thiole; Hydroxysäuren (z. B. α-Hydroxysäuren wie Milchsäure und Glykolsäure oder β-Hydroxysäuren wie Salicylsäure und Salicylsäurederivate, wie die Octanoylderivate), Phytinsäure, Liponsäure; Lysophosphatidinsäure, Haut-Peelingmittel (z. B. Phenol und dergleichen), Vitamin $B_3$-Verbindungen und Retinoide ein, die die Vorteile der vorliegenden Erfindung für die Glättung der Haut verbessern.

a) Vitamin B3-Verbindungen

Die erfindungsgemässen Zusammensetzungen können eine sichere und wirksame Menge einer Vitamin B3-Verbindung enthalten. Vitamin B3-Verbindungen sind besonders nützlich zur Regulierung des Hautzustands, wie in der gleichzeitig anhängigen US-Patentanmeldung mit dem Aktenzeichen Nr. 08/834,010 beschrieben ist, eingereicht am 11. April 1997, (entsprechend der internationalen Veröffentlichung WO 97/39733 Al, veröffentlicht am 30. Oktober 1997). Beispielhafte Derivate der genannten Vitamin $B_3$-Verbindungen schliessen Nicotinsäureester einschliesslich nicht-vasodilatierender Ester von Nicotinsäure (z. B. Tocopherylnicotinat), Nicotinylaminosäuren, Nicotinylalkoholestern von Carbonsäuren, Nicotinsäure-N-oxid und Niacinamid-N-oxid ein.

b) Retinoide

Die erfindungsgemässen Zusammensetzungen können auch ein Retinoid enthalten. "Retinoid" schliesst, wie hier verwendet, alle natürlichen und/oder synthetischen Analoga von Vitamin A oder retinolartigen Verbindungen ein, die die biologische Wirksamkeit von Vitamin A in der Haut besitzen, sowie die geometrischen Isomere und Stereoisomere dieser Verbindungen. Das Retinoid ist vorzugsweise Retinol, Retinolester (z. B. $C_2$- bis $C_{22}$-Alkylester von Retinol einschliesslich Retinylpalmitat, Retinylacetat, Retinylpropionat), Retinal und/oder Retinsäure (einschliesslich all-trans-Retinsäure und/oder 13-cis-Retinsäure), insbesondere von Retinsäure verschiedene Retinoide. Andere geeignete Retinoide sind Tocopherylretinoat [Tocopherolester von Retinsäure (trans oder cis)], Adaptalen {6-[3-(1-Adamantyl)-4-methoxyphenyl]-2-naphthoesäure} und Tazaroten (Ethyl-6-[2-(4,4-dimethylthiochroman-6-yl)-ethinyl]nicotinat). Bevorzugte Retinoide sind Retinol, Retinylpalmitat, Retinylacetat, Retinylpropionat, Retinal und Kombinationen davon. Die erfindungsgemässen Zusammensetzungen können eine sichere und wirksame Menge des Retinoids enthalten, so dass die resultierende Zusammensetzung sicher und wirksam zur Regulierung des Zustands von Horngewebe ist, vorzugsweise zur Regulierung von sichtbaren und/oder fühlbaren Diskontinuitäten der Haut, insbesondere zur Regulierung von Zeichen der Hautalterung, bevorzugter zur Regulierung von sichtbaren und/oder fühlbaren Diskontinuitäten der Hautoberflächenbeschaffenheit, die mit Hautalterung zusammenhängen.

(c) Hydroxysäuren

Die erfindungsgemässen Zusammensetzungen können eine sichere und wirksame Menge einer Hydroxysäure enthalten. Bevorzugte Hydroxysäuren zur Verwendung in den erfindungsgemässen Zusammensetzungen schliessen Salicylsäure und Salicylsäurederivate ein.

d) Peptide

Die erfindungsgemässen Zusammensetzungen könne mindestens ein zusätzliches Peptid, einschliesslich, aber nicht begrenzt auf Di-, Tri-, Tetra-, Penta- und Hexapeptide, enthalten. Solche Peptide und/oder Derivate davon

können den erfindungsgemässen Zusammensetzungen in sicheren und wirksamen Mengen zugefügt werden. "Peptide" bezieht sich hier auf sowohl die natürlich vorkommenden Peptide als auch die synthetisierten Peptide und umfasst auch Peptidmimetika und Metallkomplexe von "Peptiden". Die natürlich vorkommenden und im Handel erhältlichen Zusammensetzungen, die Peptide enthalten, sind hier auch brauchbar.

Zur Verwendung in den erfindungsgemässen Zusammensetzungen geeignete Dipeptide schliessen Carnosin (β-Ala-His) ein. Hierfür geeignete Tripeptide schliessen Gly-His-Lys, Arg-Lys-Arg und His-Gly-Gly ein. Bevorzugte Tripeptide und Derivate davon schliessen Palmitoyl-Gly-His-Lys, das als Biopeptid CL™ erworben werden kann (100 ppm Palmitoyl-Gly-His-Lys, kommerziell erhältlich von Sederma, Frankreich), Peptid CK (Arg-Lys-Arg), Peptid CK+ (ac-Arg-Lys-Arg-$NH_2$) und β-Ala-Pro-Dab-NH-Benzyl, das unter dem Namen SYN®-AKE von Pentapharm, Schweiz vertrieben wird, ein. Zur Verwendung in den erfindungsgemässen Zusammensetzungen geeignete Tetrapeptide schliessen Peptid E ein, Arg-Ser-Arg-Lys. Beispiele für geeignete Pentapeptide sind Matrixyl (Palmitoyl-Lys-Thr-Thr-Lys-Ser), erhältlich von Sederma, Frankreich, und jene, die in WO 03/037933 (Pentapharm, Schweiz) beschrieben sind. Ein zur Verwendung geeignetes Hexapeptid ist Argireline (Ac- Glu-Glu-Met-Gln-Arg-Arg-NH2), hergestellt von Lipotec, Spanien.

Die erfindungsgemässen Verbindungen sowie die kosmetischen Zusammensetzungen, die sie enthalten, werden für Hautpflegeprodukte eingesetzt, insbesondere für die Verbesserung des Hautbildes und gegen negative Auswirkungen der Hautalterung, verursacht durch den Abbau der Proteine der Basalmembran.

Die folgenden Beispiele sollen die Erfindung erläutern, ohne sie einzuschränken. Benutzte Abkürzungen im Text und in den Beispielen 1-9 bedeuten:

| | |
|---|---|
| AcOH: | Essigsäure |
| ACN: | Acetonitril |
| AK: | Antikörper |
| Ala : | Alanin |
| Arg: | Arginin |
| Asn: | Asparagin |
| Asp: | Asparaginsäure |
| Boc: | tert.-Butyloxycarbonyl |
| BSA: | Bovine Serum Albumine |
| CTR: | Chlortrityl Harz |
| Dab: | 2,4-Diaminobuttersäure |
| Dap: | 2,3-Diaminopropionsäure |
| DBU: | 1,8-Diazabicyclo[5,9,0]undec-7-ene(1,5-5) |
| DCC: | N,N'-Dicyclohexylcarbodiimid |
| DCM: | Dichlormethan |
| DIC: | N,N'-Diisopropylcarbodiimid |
| DIPEA: | Diisopropylethylamin |
| DMEM: | Dulbecco's Modified Eagle Medium |
| DMF: | Dimethylformamid |
| EM: | Elektronenmikroskop |
| EtOAc: | Essigsäureethylester |
| FCS: | Foetal Calf Serum |
| Fmoc: | 9-Fluorenylmethyloxycarbonyl |
| GF/A: | Glasfaser-Mikrofilter |
| Gln: | Glutamin |
| Glu: | Glutaminsäure |
| HaCaT: | Humane immortalisierte Keratinozyten |
| HOBt: | 1-Hydroxybenzotriazol |
| ILe: | Isoleucin |
| LH20: | Amersham size exclusion resin |
| Lys: | Lysin |
| Met: | Methionin |
| MS: | Massenspektrometrie |
| NMM: | N-Methylmorpholin |
| NMR: | Magnetische Kernspin Resonanz |
| Nle: | Norleucin |
| Orn: | Ornithin |
| Palm: | Palmitoyl |
| PBS: | Phosphate buffered saline |

| | |
|---|---|
| PE: | Petrolether |
| Phe: | Phenylalanin |
| RT: | Raumtemperatur |
| Ser: | Serin |
| tBu: | tert.-Butyl |
| TBTU: | O-(Benzotriazol-1-yl)-N,N,N',N',- tetramethyluronium tetrafluoroborat |
| TCTU: | O-(1H-6-Chlorobenzotriazol-1-yl)-1,1,3,3- tetramethyluronium hexafluorphosphat |
| TGF-β1/2: | Transforming Growth Factor-β1 resp. -β2 |
| TFA: | Trifluoressigsäure |
| THF: | Tetrahydrofuran |
| Thr: | Threonin |
| Tris: | Tris-(hydroxymethyl)-aminomethan |
| Tween-20: | Polyethylenglycol-sorbitan-monolaurat-Lösung |
| Val: | Valin |
| Z: | Benzyloxycarbonyl |

Beispiel 1 (Bestimmung der Stimulierung der Laminin V-Synthese in Keratinocyten-Zellkulturen der Zellinie HaCaT durch Behandlung mit den erfindungsgemässen Peptid- Derivaten

Die Laminin V Produktion pro Zelle von in-vitro kultivierten HaCaT Keratinocyten wurde mittels eines ELISA (Enzyme-Linked Immunosorbent Assay) detektiert. In Gegenwart der peptidischen Wirkstoffe wurde die Steigerung der Laminin V-Produktion der Zellen mit dieser Methode quantifiziert. Die humanen HaCaT Keratinocyten waren ein Geschenk von Prof. Fusenig vom Deutschen Krebsforschungszentrum in Heidelberg und wurden in Kulturmedium nach Standard-Zellkulturmethoden gezüchtet. Nach 72 Stunden Inkubationszeit mit den entsprechenden Peptiden (Wirkstoff) erfolgt die quantitative Bestimmung mit einem für Laminin V spezifischen Antikörper. Nach Bestimmung des Laminin V Gehaltes wird die Zellzahl mittels des CyQUANT® der Firma Molecular Probes ermittelt. Aus den Einzelwerten wird der Laminin V-Gehalt pro Zelle als Units berechnet.

Material:

| Kulturmedium: | Testmedium: |
|---|---|
| - DMEM<br>- 10% FCS<br>- 100IU/ml Penicillin<br>- 0.1mg/ml Streptomycin | - DMEM<br>- kein FCS<br>- 100IU/ml Penicillin<br>- 0.1mg/ml Streptomycin |

| Waschpuffer: | Milchlösung: |
|---|---|
| - 0.05M Tris, pH 8.5<br>- 0.15M NaCl<br>- 0.1 % BSA<br>- 0.1 % Tween-20 | - Waschpuffer<br>- 5% Milchpulver |

| AK-Verdünnungslösung: | Substratlösung: |
|---|---|
| - 50ml SuperBlock (37515; Pierce) Tablet (34006; ierce)P | - 1 ImmunoPure® OPD |
| - 450ml $H_2O$ | - 9ml $H_2O$ |
| - 0.05% Tween | - 1ml Stable Peroxide Substr. Buffer, 10x (34062; Pierce) |

1. AK (P3H9-2; Santa Cruz Biotechnology, Inc.) wird 1/60'000 und 2. AK (31430; Socochim S.A.) 1/500 mit AK-Verd.Lsg verdünnt.

Methode:

Die Keratinocyten werden mit einer Dichte von ca. 5000 Zellen/Well in 96well-plates ausgesät für 3 Tage bis zur Konfluenz im Kulturmedium inkubiert (37 °C / 10% $CO_{2)}$. Das Medium wird gegen Testmedium mit drei unterschiedlichen Konzentrationen in triplicate an Testsubstanz ausgetauscht. Folgende Kontrollen werden auf jeder Platte mitgetestet:

| Negativkontrollen: | Positivkontrollen: |
|---|---|
| A) | A) |
| - mit Zellen | - mit Zellen |
| - ohne 1.AK; mit 2.AK | - mit 1. und 2. AK |
| B) | B) |
| - ohne Zellen | - mit Zellen |
| - mit 1. und 2. AK | - mit 1. und 2. AK |
| | - mit 10ng/ml TGF-β2 |

C) Für jedes Peptid wird ein well ohne Zellen getestet, um die unspezifische Bindung der beiden AK auszuschließen.

Die Platten werden für weitere 72 Stunden inkubiert. Nach Abschluss dieser Inkubationszeit wird das abgelagerte Laminin V nach folgendem Protokoll detektiert und quantifiziert:

- ■ Medium verwerfen und mit 200µl/well PBS waschen
- ■ mit 100µl/well Methanol fixieren -> 15min/ RT/ Shaker 600rpm
- ■ Methanol verwerfen und mit 200µl/well Milchlösung blockieren -> 30min/ RT/ Shaker 600rpm
- ■ Milchlösung verwerfen und mit 100µl/well 1.AK-Verd. inkubieren -> 2h/ RT/ Shaker 600rpm
- ■ 1.AK-Verd. verwerfen und 3x mit 200µl/well Waschpuffer waschen
- ■ mit 100µl/well 2.AK-Verd. inkubieren -> 3h/ RT/ Shaker 600rpm
- ■ 2.AK-Verd. verwerfen; 3x mit 200µl/well Waschpuffer und 1x 100µl/well PBS waschen
- ■ add 100µl/well Substratlösung -> 15min/ RT/ Shaker 600rpm
- ■ mit 50µl/well $H_2SO_4$ (2M) Reaktion stoppen und bei 492nm messen.
- ■ Die Färbelösung wird verworfen, die Platte mit $H_2O$ bidest. gewaschen und für ca. 16 Stunden bei -80°C eingefroren.
- ■ Die Platte wird aufgetaut und die Zellzahl mittels des CyQUANT Assays nach Herstellervorschrift gemessen.

Die Laminin V Produktion pro Zelle wird nach folgender Formel berechnet: (Wert $OD_{Laminin}$ / Wert $RFU_{Zellzahl}$) x 100 Die berechneten Werte sind willkürliche Units

Tabelle 2: Stimulation von Laminin V im ELISA:

| Nr | Substanz | Konz [µmol/L] | %Stimulation in Relation zu Kontrolle |
|---|---|---|---|
| | TGF-β (Positivkontrolle) | 10 ng/ml | 40-90 |
| 1.3 | Palm-Lys-Val-Dab-OH | 25<br>50<br>100 | 34-71<br>48-75<br>64-83 |
| 1.10 | Palm-Lys-Val-Dab-NH-Octyl | 2.5<br>5.0<br>10.0 | 40-45<br>154-200<br>772-940 |
| 1.11 | Palm-Lys-Val-Dab-N(Octyl)2 | 0.25<br>0.5<br>1.0 | 15-31<br>28-31<br>32-75 |

Beispiel 2: Bestimmung der Stimulierung der Collagen IV-Synthese in Keratinocyten-Zellkulturen der Zellinie HaCaT durch Behandlung mit den erfindungsgemässen Peptid Derivaten

Die Collagen IV-Produktion pro Zelle von in-vitro kultivierten HaCaT Keratinocyten wurde mittels eines ELISA (Enzyme-Linked Immunosorbent Assay) detektiert. In Gegenwart der peptidischen Wirkstoffe wurde die Steigerung der Collagen-IV-Produktion der Zellen mit dieser Methode quantifiziert. Die humanen HaCaT Keratinocyten waren ein Geschenk von Prof. Fusenig vom Deutschen Krebsforschungszentrum in Heidelberg und wurden in Kulturmedium nach Standard-Zellkulturmethoden gezüchtet. Nach 72 Stunden Inkubationszeit mit den entsprechenden Peptiden (Wirkstoffe), erfolgt die quantitative Bestimmung mit einem für Collagen IV spezifischen Antikörper. Nach Bestimmung des Collagen IV Gehaltes wird die Zellzahl mittels des CyQUANT® der Firma Molecular Probes ermittelt. Aus den Einzelwerten wird der Collagen IV-Gehalt pro Zelle als Units berechnet.

Material:

| Kulturmedium: | Testmedium: |
|---|---|
| - DMEM<br>- 10% FCS<br>- 100IU/ml Penicillin<br>- 0.1mg/ml Streptomycin | - DMEM<br>- kein FCS<br>- 100IU/ml Penicillin<br>- 0.1mg/ml Streptomycin |

| Waschpuffer: | Milchlösung: |
|---|---|
| - 0.05M Tris, pH 8.5<br>- 0.15M NaCl<br>- 0.1 % BSA<br>- 0.1 % Tween-20 | - Waschpuffer<br>- 5% Milchpulver |

| AK-Verdünnungslösung: | Substratlösung: |
|---|---|
| - 50ml SuperBlock (37515; Pierce) Tablet (34006; Pierce) | - 1 ImmunoPure® OPD |
| - 450ml $H_2O$ | - 9ml $H_2O$ |
| - 0.05% Tween Buffer, 10x (34062; Pierce) | - 1ml Stable Peroxide Substr. |

1. AK (H-234; Santa Cruz Biotechnology, Inc.) wird 1/200 und 2. AK (31460; Socochim S.A.) 1/500 mit AK-Verd.Lsg verdünnt.

Methode:

Die Keratinocyten werden mit einer Dichte von ca. 5000 Zellen/Well in 96well-plates ausgesät für 3 Tage bis zur Konfluenz im Kulturmedium inkubiert (37°C / 10% $CO_2$). Das Medium wird gegen Testmedium mit drei unterschiedlichen Konzentrationen in triplicate an Testsubstanz ausgetauscht. Folgende Kontrollen werden auf jeder Platte mitgetestet:

| Negativkontrollen: | Positivkontrollen: |
|---|---|
| A)<br>- mit Zellen<br>- ohne 1.AK; mit 2.AK | A)<br>- mit Zellen<br>- mit 1. und 2. AK |
| B)<br>- ohne Zellen<br>- mit 1. und 2. AK | B)<br>- mit Zellen<br>- mit 1. und 2. AK<br>- mit 10ng/ml TGF-β2 |

C) Für jedes Peptid wird ein well ohne Zellen getestet, um die unspezifische Bindung der beiden AK auszuschließen.

Die Platten werden für weitere 72 Stunden inkubiert. Nach Abschluss dieser Inkubationszeit wird das abgelagerte Collagen IV nach folgendem Protokoll detektiert und quantifiziert:

- Medium verwerfen und mit 200µl/well PBS waschen
- mit 100µl/well Methanol fixieren -> 15min/ RT/ Shaker 600rpm
- Methanol verwerfen und mit 200µl/well Milchlösung blockieren -> 30min/ RT/ Shaker 600rpm
- Milchlösung verwerfen und mit 100µl/well 1.AK-Verd. inkubieren -> 2h/ RT/ Shaker 600rpm
- 1.AK-Verd. verwerfen und 3x mit 200µl/well Waschpuffer waschen
- mit 100µl/well 2.AK-Verd. inkubieren -> 3h/ RT/ Shaker 600rpm
- 2.AK-Verd. verwerfen; 3x mit 200µl/well Waschpuffer und 1x 100µl/well PBS waschen
- add 100µl/well Substratlösung -> 15min/ RT/ Shaker 600rpm
- mit 50µl/well $H_2SO_4$ (2M) Reaktion stoppen und bei 492nm messen.
- Die Färbelösung wird verworfen, die Platte mit $H_2O$ bidest. gewaschen und für ca. 16 Stunden bei -80°C eingefroren.
- Die Platte wird aufgetaut und die Zellzahl mittels des CyQUANT Assays nach Herstellervorschrift gemessen.
- Die Collagen IV Produktion pro Zelle wird nach folgender Formel berechnet:

$$(\text{Wert OD}_{\text{Collagen}}\ \text{IV} / \text{Wert RFU}_{\text{Zellzahl}}) \times 100$$

Die berechneten Werte sind willkürliche Units.
Dabei zeigen die Verbindungen 1.1, 1.10, 1.11 und 2.5-2.7 aus der Tabelle 1 eine gute bis sehr gute stimulierende Wirkung.

Beispiel 3: Bestimmung der Stimulierung der Collagen VII Synthese in Keratinocyten-Zellkulturen der Zellinie HaCaT durch Behandlung mit den erfindungsgemässen Peptid Derivaten

Die Collagen VII Produktion pro Zelle von in-vitro kultivierten HaCaT Keratinocyten wurde mittels eines ELISA (Enzyme-Linked Immunosorbent Assay) detektiert. In Gegenwart der peptidischen Wirkstoffe wurde die Steigerung der Collagen VII-Produktion der Zellen mit dieser Methode quantifiziert. Die humanen HaCaT Keratinocyten waren ein Geschenk von Prof. Fusenig vom Deutschen Krebsforschungszentrum in Heidelberg und wurden in Kulturmedium nach Standard-Zellkulturmethoden gezüchtet. Nach 72 Stunden Inkubationszeit mit den entsprechenden Peptiden (Wirkstoffe), erfolgt die quantitative Bestimmung mit einem für Collagen VII spezifischen Antikörper. Nach Bestimmung des Collagen VII Gehaltes wird die Zellzahl mittels des CyQUANT® der Firma Molecular Probes ermittelt. Aus den Einzelwerten wird der Collagen VII-Gehalt pro Zelle als Units berechnet.

Material:

| Kulturmedium: | Testmedium: |
|---|---|
| - DMEM | - DMEM |
| - 10% FCS | - kein FCS |
| - 100IU/ml Penicillin | - 100IU/ml Penicillin |
| - 0.1mg/ml Streptomycin | - 0.1mg/ml Streptomycin |

| Waschpuffer: | Milchlösung: |
|---|---|
| - 0.05M Tris, pH 8.5 | - Waschpuffer |
| - 0.15M NaCl | - 5% Milchpulver |
| - 0.1 % BSA | |
| - 0.1 % Tween-20 | |

(fortgesetzt)

| AK-Verdünnungslösung: | Substratlösung: |
|---|---|
| - 50ml SuperBlock (37515; Pierce) Tablet (34006; Pierce) | - 1 ImmunoPure® OPD |
| - 450ml $H_2O$ | - 9ml $H_2O$ |
| - 0.05% Tween Buffer, 10x (34062; Pierce) | - 1ml Stable Peroxide Substr. |

1. AK (C-16; Santa Cruz Biotechnology, Inc.) wird 1/200 und 2. AK (31402; Socochim S.A.) 1/500 mit AK-Verd.Lsg verdünnt.

Methode:

Die Keratinocyten werden mit einer Dichte von ca. 5000 Zellen/Well in 96well-plates ausgesät für 3 Tage bis zur Konfluenz im Kulturmedium inkubiert (37 °C / 10% $CO_2$). Das Medium wird gegen Testmedium mit drei unterschiedlichen Konzentrationen in triplicate an Testsubstanz ausgetauscht. Folgende Kontrollen werden auf jeder Platte mitgetestet:

| Negativkontrollen: | Positivkontrollen: |
|---|---|
| A) | A) |
| - mit Zellen | - mit Zellen |
| - ohne 1.AK; mit 2.AK | - mit 1. und 2. AK |
| B) | B) |
| - ohne Zellen | - mit Zellen |
| - mit 1. und 2. AK | - mit 1. und 2. AK |
| | - mit 10ng/ml TGF-β2 |

C) Für jedes Peptid wird ein well ohne Zellen getestet, um die unspezifische Bindung der beiden AK auszuschließen.

Die Platten werden für weitere 72 Stunden inkubiert. Nach Abschluss dieser Inkubationszeit wird das abgelagerte Collagen VII nach folgendem Protokoll detektiert und quantifiziert:

- ■ Medium verwerfen und mit 200µl/well PBS waschen
- ■ mit 100µl/well Methanol fixieren -> 15min/ RT/ Shaker 600rpm
- ■ Methanol verwerfen und mit 200µl/well Milchlösung blockieren -> 30min/ RT/ Shaker 600rpm
- ■ Milchlösung verwerfen und mit 100µl/well 1.AK-Verd. inkubieren -> 2h/ RT/ Shaker 600rpm
- ■ 1.AK-Verd. verwerfen und 3x mit 200µl/well Waschpuffer waschen
- ■ mit 100µl/well 2.AK-Verd. inkubieren -> 3h/ RT/ Shaker 600rpm
- ■ 2.AK-Verd. verwerfen; 3x mit 200µl/well Waschpuffer und 1x 100µl/well PBS waschen
- ■ add 100µl/well Substratlösung -> 15min/ RT/ Shaker 600rpm
- ■ mit 50µl/well $H_2SO_4$ (2M) Reaktion stoppen und bei 492nm messen.
- ■ Die Färbelösung wird verworfen, die Platte mit $H_2O$ bidest. gewaschen und für ca. 16 Stunden bei -80°C eingefroren.
- ■ Die Platte wird aufgetaut und die Zellzahl mittels des CyQUANT Assays nach Herstellervorschrift gemessen.
- ■ Die Collagen VII Produktion pro Zelle wird nach folgender Formel berechnet:

$$(\text{Wert OD}_{\text{Collagen}} \text{ VII} / \text{Wert RFU}_{\text{Zellzahl}}) \times 100$$

Die berechneten Werte sind willkürliche Units.
Dabei zeigen die Verbindungen 1.10 und 2.5-2.7 der Tabelle 1 eine gute bis sehr gute stimulierende Wirkung.

Beispiel 4: Bestimmung der Stimulierung der Integrin beta4 Synthese in Keratinocyten-Zellkulturen der Zellinie HaCaT durch Behandlung mit den erfindungsgemässen Peptid Derivaten

Die Integrin beta4 Produktion pro Zelle von in-vitro kultivierten HaCaT Keratinocyten wurde mittels eines ELISA (Enzyme-Linked Immunosorbent Assay) detektiert. In Gegenwart der peptidischen Wirkstoffe wurde die Steigerung der Integrin beta4 -Produktion der Zellen mit dieser Methode quantifiziert. Die humanen HaCaT Keratinocyten waren ein Geschenk von Prof. Fusenig vom Deutschen Krebsforschungszentrum in Heidelberg und wurden in Kulturmedium nach Standard-Zellkulturmethoden gezüchtet. Nach 72 Stunden Inkubationszeit mit den entsprechenden Peptiden (Wirkstoff), erfolgt die quantitative Bestimmung mit einem für Integrin beta4 spezifischen Antikörper. Nach Bestimmung des Integrin beta4 Gehaltes wird die Zellzahl mittels des CyQUANT® der Firma Molecular Probes ermittelt. Aus den Einzelwerten wird der Integrin beta4-Gehalt pro Zelle als Units berechnet.

Material:

| Kulturmedium: | Testmedium: |
|---|---|
| - DMEM<br>- 10% FCS<br>- 100IU/ml Penicillin<br>- 0.1mg/ml Streptomycin | - DMEM<br>- kein FCS<br>- 100IU/ml Penicillin<br>- 0.1mg/ml Streptomycin |

| Waschpuffer: | Milchlösung: |
|---|---|
| - 0.05M Tris, pH 8.5<br>- 0.15M NaCl<br>- 0.1 % BSA<br>- 0.1 % Tween-20 | - Waschpuffer<br>- 5% Milchpulver |

| AK-Verdünnungslösung: | Substratlösung: |
|---|---|
| - 50ml SuperBlock (37515; Pierce) Tablet (34006; Pierce) | - 1 ImmunoPure® OPD |
| - 450ml $H_2O$ | - 9ml $H_2O$ |
| - 0.05% Tween Buffer, 10x (34062; Pierce) | - 1ml Stable Peroxide Substr. |

1. AK (A9; Santa Cruz Biotechnology, Inc.) wird 1/200 und 2. AK (31430; Socochim S.A.) 1/500 mit AK-Verd.Lsg verdünnt.

Methode:

Die Keratinocyten werden mit einer Dichte von ca. 5000 Zellen/Well in 96well-plates ausgesät für 3 Tage bis zur Konfluenz im Kulturmedium inkubiert (37°C / 10% $CO_2$). Das Medium wird gegen Testmedium mit drei unterschiedlichen Konzentrationen in triplicate an Testsubstanz ausgetauscht. Folgende Kontrollen werden auf jeder Platte mitgetestet:

| Negativkontrollen: | Positivkontrollen: |
|---|---|
| A) | A) |
| - mit Zellen | - mit Zellen |
| - ohne 1.AK; mit 2.AK | - mit 1. und 2. AK |
| B) | B) |
| - ohne Zellen | - mit Zellen |
| - mit 1. und 2. AK | - mit 1. und 2. AK |
| | - mit 10ng/ml TGF-β2 |

C) Für jedes Peptid wird ein well ohne Zellen getestet, um die unspezifische Bindung der beiden AK auszuschließen.

Die Platten werden für weitere 72 Stunden inkubiert. Nach Abschluss dieser Inkubationszeit wird das abgelagerte Integrin β4 nach folgendem Protokoll detektiert und quantifiziert:

- Medium verwerfen und mit 200µl/well PBS waschen
- mit 100µl/well Methanol fixieren -> 15min/ RT/ Shaker 600rpm
- Methanol verwerfen und mit 200µl/well Milchlösung blockieren -> 30min/ RT/ Shaker 600rpm
- Milchlösung verwerfen und mit 100µl/well 1.AK-Verd. inkubieren -> 2h/ RT/ Shaker 600rpm
- 1.AK-Verd. verwerfen und 3x mit 200µl/well Waschpuffer waschen
- mit 100µl/well 2.AK-Verd. inkubieren -> 3h/ RT/ Shaker 600rpm
- 2.AK-Verd. verwerfen; 3x mit 200µl/well Waschpuffer und 1x 100µl/well PBS waschen
- add 100µl/well Substratlösung -> 15min/ RT/ Shaker 600rpm
- mit 50µl/well $H_2SO_4$ (2M) Reaktion stoppen und bei 492nm messen.
- Die Färbelösung wird verworfen, die Platte mit $H_2O$ bidest. gewaschen und für ca. 16 Stunden bei -80°C eingefroren.
- Die Platte wird aufgetaut und die Zellzahl mittels des CyQUANT Assays nach Herstellervorschrift gemessen.
- Die Integrin β4 Produktion pro Zelle wird nach folgender Formel berechnet:

$$(\text{Wert OD}_{\text{Integrin } \beta 4} \ / \ \text{Wert RFU}_{\text{Zellzahl}}) \times 100$$

Die berechneten Werte sind willkürliche Units.
Dabei zeigen die Verbindungen 1.1 und 2.5-2.7 der Tabelle 1 eine gute bis sehr gute stimulierende Wirkung.

<u>Beispiel 5:</u> Bestimmung der Stimulierung der Collagen XVII Synthese in Keratinocyten-Zellkulturen der Zellinie HaCaT durch Behandlung mit den erfindungsgemässen Peptid Derivaten

Die Collagen XVII Produktion pro Zelle von in-vitro kultivierten HaCaT Keratinocyten wurde mittels eines ELISA (Enzyme-Linked Immunosorbent Assay) detektiert. In Gegenwart der peptidischen Wirkstoffe wurde die Steigerung der Collagen XVII-Produktion der Zellen mit dieser Methode quantifiziert. Die humanen HaCaT Keratinocyten waren ein Geschenk von Prof. Fusenig vom Deutschen Krebsforschungszentrum in Heidelberg und wurden in Kulturmedium nach Standard-Zellkulturmethoden gezüchtet. Nach 72 Stunden Inkubationszeit mit den entsprechenden Peptiden (Wirkstoffe), erfolgt die quantitative Bestimmung mit einem für Collagen XVII spezifischen Antikörper. Nach Bestimmung des Collagen XVII Gehaltes wird die Zellzahl mittels des CyQUANT® der Firma Molecular Probes ermittelt. Aus den Einzelwerten wird der Collagen XVII-Gehalt pro Zelle als Units berechnet.

Material:

| Kulturmedium: | Testmedium: |
|---|---|
| - DMEM | - DMEM |
| - 10% FCS | - kein FCS |
| - 100IU/ml Penicillin | - 100IU/ml Penicillin |
| - 0.1mg/ml Streptomycin | - 0.1mg/ml Streptomycin |

| Waschpuffer: | Milchlösung: |
|---|---|
| - 0.05M Tris, pH 8.5 | - Waschpuffer |
| - 0.15M NaCl | - 5% Milchpulver |
| - 0.1 % BSA | |
| - 0.1 % Tween-20 | |

(fortgesetzt)

| AK-Verdünnungslösung: | Substratlösung: |
|---|---|
| - 50ml SuperBlock (37515; Pierce) | - 1 ImmunoPure® OPD Tablet (34006; Pierce) |
| - 450ml $H_2O$ | - 9ml $H_2O$ |
| - 0.05% Tween | - 1ml Stable Peroxide Substr. Buffer, 10x (34062; Pierce) |

1. AK (STO-115; Davids Biotechnologie) wird 1/200 und 2. AK (31430; Socochim S.A.) 1/500 mit AK-Verd.Lsg verdünnt.

Methode:

Die Keratinocyten werden mit einer Dichte von ca. 5000 Zellen/Well in 96well-plates ausgesät für 3 Tage bis zur Konfluenz im Kulturmedium inkubiert (37°C / 10% $CO_2$). Das Medium wird gegen Testmedium mit drei unterschiedlichen Konzentrationen in triplicate an Testsubstanz ausgetauscht. Folgende Kontrollen werden auf jeder Platte mitgetestet :

| Negativkontrollen: | Positivkontrollen: |
|---|---|
| A) | A) |
| - mit Zellen | - mit Zellen |
| - ohne 1.AK; mit 2.AK | - mit 1. und 2. AK |
| B) | B) |
| - ohne Zellen | - mit Zellen |
| - mit 1. und 2. AK | - mit 1. und 2. AK |
| | - mit 10ng/ml TGF-β2 |

C) Für jedes Peptid wird ein well ohne Zellen getestet, um die unspezifische Bindung der beiden AK auszuschließen.

Die Platten werden für weitere 72 Stunden inkubiert. Nach Abschluss dieser Inkubationszeit wird das abgelagerte Collagen XVII nach folgendem Protokoll detektiert und quantifiziert:

- Medium verwerfen und mit 200µl/well PBS waschen
- mit 100µl/well Methanol fixieren -> 15min/ RT/ Shaker 600rpm
- Methanol verwerfen und mit 200µl/well Milchlösung blockieren -> 30min/ RT/ Shaker 600rpm
- Milchlösung verwerfen und mit 100µl/well 1.AK-Verd. inkubieren -> 2h/ RT/ Shaker 600rpm
- 1.AK-Verd. verwerfen und 3x mit 200µl/well Waschpuffer waschen
- mit 100µl/well 2.AK-Verd. inkubieren -> 3h/ RT/ Shaker 600rpm
- 2.AK-Verd. verwerfen; 3x mit 200µl/well Waschpuffer und 1x 100µl/well PBS waschen
- add 100µl/well Substratlösung -> 15min/ RT/ Shaker 600rpm
- mit 50µl/well $H_2SO_4$ (2M) Reaktion stoppen und bei 492nm messen.
- Die Färbelösung wird verworfen, die Platte mit $H_2O$ bidest. gewaschen und für ca. 16 Stunden bei -80°C eingefroren.
- Die Platte wird aufgetaut und die Zellzahl mittels des CyQUANT Assays nach Herstellervorschrift gemessen.
- Die Collagen XVII Produktion pro Zelle wird nach folgender Formel berechnet:

$$(\text{Wert}\ _{\text{ODCollagen XVII}}\ /\ \text{Wert RFU}_{\text{Zellzahl}})\ \times\ 100$$

Die berechneten Werte sind willkürliche Units.
Dabei zeigen die Verbindungen 1.1, 1.10, 1.11 und 2.5-2.7 eine gute bis sehr gute stimulierende Wirkung.

Beispiel 6: Formulierung einer Salbe

Prozedur: Die Inhaltstoffe 1-5 (A) werden auf 70°C erhitzt. Die Inhaltstoffe 6-7 (B) werden auf 75°C erhitzt. Unter Rühren wird B zu A gegeben, auf 50°C gekühlt, homogenisiert und auf 30°C abgekühlt. Dann werden die Inhaltstoffe 8 und 9 (C) und die Inhaltsstoffe 10 und 11 (D) nacheinander hinzugegeben und kalt gerührt.

| Nummer | Inhaltsstoff | | % w/w |
|---|---|---|---|
| 1 | (A) | Tego Care 450 | 3.00 |
| 2 | | Cetearylalkohol | 2.25 |
| 3 | | Glycerylstearat | 2.25 |
| 4 | | Cetiol 868 | 10.00 |
| 5 | | Squalane | 5.00 |
| 6 | (B) | Deionisiertes Wasser | 66.995 |
| 7 | | Natriumhyaluronat | 5.00 |
| 8 | (C) | Glycerin | 5.00 |
| 9 | | Phenonip | 0.5 |
| 10 | (D) | Palm-Lys-Val-Dab-OH (1.3) | 0.0025 |
| 11 | | Palm-Lys-Val-Dab-Thr-OH (2.7) | 0.0025 |

Beispiel 7: Formulierung eines Gels

Prozedur: Die Inhaltstoffe 2-6 (A) werden nacheinander in deionisiertem Wasser gelöst. Mit Inhaltstoff 7 (B) wird auf pH 6.0 gestellt. Dann werden Inhaltstoffe 8 und 9 (C) hinzugegeben.

| Nummer | Inhaltsstoff | | % w/w |
|---|---|---|---|
| 1 | (A) | Deionisiertes Wasser | 92.095 |
| 2 | | 1,3-Butandiol | 5.00 |
| 3 | | Phenonip | 0.50 |
| 4 | | Abil B 8843 | 1.50 |
| 5 | | Carboxymethyl Cellulose | 0.15 |
| 6 | | Carbopol Ultrez 10 | 0.75 |
| 7 | (B) | NaOH | |
| 8 | (C) | Palm-Lys-Val-Dab-OH (1.3) | 0.0025 |
| 9 | | Palm-Lys-Val-Dab-Thr-OH (2.7) | 0.0025 |

Beispiel 8: Herstellung von erfindungsgemässen Verbindungen der Formel (I) worin X -$NR^2$- bedeutet und sowohl $R^1$ als auch $R^2$ von H verschieden sind, und von erfindungsgemässen Verbindungen entsprechend der Formel (I), worin aber $XR^1$ mit X in der Bedeutungsmöglichkeit -NH- den Rest einer alpha-Aminosäure bedeutet, und von Salzen solcher Verbindungen

Die Analyse der gemäss diesem Beispiel erhaltenen Eluate und Produkte wurde mit Proton-NMR, HPLC-Elektrospray-MS oder Elementaranalyse ausgeführt. Die Verbindungen können nach den im Folgenden beschriebenen an sich bekannten Verfahren (allgemeinen Vorschriften von M. Bodanszky "The Practice of Peptide Synthesis" Springer Verlag, 2nd Edition 1994) hergestellt werden. Entsprechend wird die Aminosäure, beispielsweise Lysin, in einer Festphasensynthese am carboxyterminalen Ende an ein Harz angeknüpft, wobei deren Aminogruppe durch eine Schutzgruppe, z.B. durch die Fmoc-Schutzgruppe, geschützt wird. Die Seitenkette wird z.B. mit Boc oder t-Butyl geschützt. Die Schutzgruppen werden nach Bedarf selektiv abgespalten, um die weiteren Aminosäure Derivate mit den in der

Peptidsynthese üblichen Reagenzien anzuknüpfen bis die gewünschte Sequenz vollständig aufgebaut ist. Das Peptid wird dann am carboxyterminalen Ende vom Harz abgespalten und das rohe Peptid durch Eintropfen in ein geeignetes Lösungsmittelgemisch ausgefällt. Das Gemisch wird über HPLC gereinigt, ggf. in die Gegenionen ausgetauscht und die Substanz lyophylisiert.

Beispiel 8.1: Herstellung von Palm-Lys(Boc)-Val-Dab(Boc)-Thr(tBu)-CTR an der Festphase

An 1.75g (Beladung: 0.8 mmol/g) 2-Chlortritylchlorid-Harz werden durch sukzessive Peptidverknüpfungen die geschützte Aminosäuren Fmoc-Thr(tBu)-OH, Fmoc-Dab(Boc)-OH, Fmoc-Val-OH, Fmoc-Lys(Boc)-OH und Palmitinsäure verknüpft und.somit das geschützte Peptid aufgebaut.

Beispiel 8.2: Herstellung von Palm-Lys-Val-Dab-Thr-OH • 2TFA an der Festphase

Das Peptid wird mittels 30 minütiger Behandlung mit 10 ml 95% TFA vom Harz abgespalten. Das Harz wird abfiltriert und die Lösung in 100 ml $Et_2O$ eingetropft. Der gebildete Niederschlag wird abgesaugt, gewaschen und nach dem Trocknen mit Hilfe von präparativer HPLC gereinigt und anschliessend lyophilisiert. Man erhält 391mg (34%) eines farblosen Pulvers. Die theoretische Masse von 686 wurde mit einem Befund von 687 bestätigt.

Beispiel 8.3: Herstellung von erfindungsgemässen Verbindungen der Formel (I), worin X -$NR^2$- bedeutet und sowohl $R^1$ als auch $R^2$ von H verschieden sind

Solche Verbindungen können in Analogie zu dem in Beispiel 7 von WO 2004/099237 Al beschriebenen Verfahren hergestellt werden.

## Patentansprüche

**1.** Zusammensetzung, insbesondere eine topisch applizierbare Zusammensetzung, enthaltend mindestens eine Verbindung der allgemeinen Formel (I),

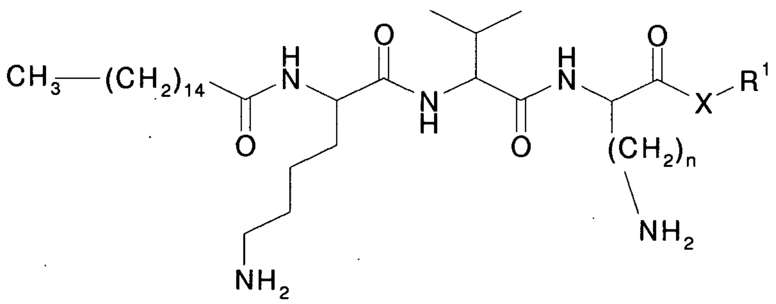

(I)

worin
$R^1$ H, $C_1$-$C_{20}$-Alkyl, Cycloalkyl mit höchstens 8 C oder Aryl-$C_1$-$C_4$-Alkyl mit höchstens 10 C im Arylteil,
n 1-4,
X -O-, -NH- oder -$NR^2$- und
$R^2$ H oder $C_1$-$C_{20}$-Alkyl bedeuten;
sowie mindestens eine Verbindung entsprechend der obigen Formel (I), worin aber
$XR^1$ mit X in der Bedeutungsmöglichkeit -NH- den Rest einer alpha-Aminosäure bedeutet.

**2.** Zusammensetzung nach Anspruch 1, **dadurch gekennzeichnet, dass** die in Anspruch 1 definierten Verbindungen

in Form von dermatologisch verträglichen Salzen vorliegen.

3. Zusammensetzung nach Anspruch 1 oder 2, **dadurch gekennzeichnet, dass** in den in Anspruch 1 definierten Verbindungen die Aminosäurereste als Racemate oder in ihrer enantiomerenreinen L und D Form vorliegen.

4. Zusammensetzung nach einem der Ansprüche 1 bis 3, **dadurch gekennzeichnet, dass** eine Verbindung der in Anspruch 1 definierten Formel (I), worin $R^1$ H oder $C_1$-$C_{20}$-Alkyl, n 2 oder 4 und X -O- bedeuten, mit einer Verbindungen entsprechend der in Anspruch 1 definierten allgemeinen Formel (I), worin aber $XR^1$ mit X in der Bedeutungsmöglichkeit -NH- den Rest einer natürlichen alpha-Aminosäure bedeutet und n 2 bedeutet, kombiniert werden.

5. Zusammensetzung nach Anspruch 4, **dadurch gekennzeichnet, dass** die Kombinationspartner Palm-Lys-Val-Dab-OH und Palm-Lys-Val-Dab-Thr-OH sind.

6. Zusammensetzung nach einem der Ansprüche 1 bis 5, **dadurch gekennzeichnet, dass** diese die in diesen Ansprüchen erwähnten Verbindungen in einer Konzentration im Bereich von 0,5 bis 5000 ppm (w/w), vorzugsweise im Bereich von 1 bis 1000 ppm (w/w), enthält.

7. Zusammensetzung, insbesondere eine topisch applizierbare Zusammensetzung, gemäss einem der Ansprüche 1 bis 6, **dadurch gekennzeichnet, dass** sie zusätzlich eine sichere und wirksame Menge von mindestens einem weiteren Hautpflegewirkstoff, ausgewählt aus der Gruppe bestehend aus Wirkstoffen für die Abschuppung, Anti-Aknewirkstoffen, Vitamin B3-Verbindungen, Retinoiden, Di-, Tri-, Tetra-, Penta- und Hexapeptiden und Derivaten davon, Hydroxysäuren, Radikalfängern, entzündungshemmenden Mitteln, Hautbräunungswirkstoffen, Hautaufhellungsmitteln, Anti-Cellulitismitteln, Flavonoiden, antimikrobiellen Wirkstoffen, Hautheilungsmitteln, antimykotischen Wirkstoffen, Sonnenschutzwirkstoffen, Farnesol, Phytantriol, Allantoin, Glucosamin und Mischungen davon enthält.

8. Zusammensetzung nach einem der Ansprüche 1 bis 7 **dadurch gekennzeichnet, dass** sie zusätzlich einen dermatologischen Träger enthält.

9. Zusammensetzung nach einem der Ansprüche 1 bis 8, **dadurch gekennzeichnet, dass** diese die wirksamen Verbindungen in Lösung, als Dispersion, als Emulsion oder eingekapselt in Träger, vorzugsweise als Makro-, Mikro- oder Nanokapseln, in Liposomen oder Chylomikronen, oder eingeschlossen in Makro-, Mikro- oder Nanoteilchen oder in Mikroschwämme oder absorbiert auf pulverförmigen organischen Polymeren, Talk, Bentonit und verwandten mineralischen Trägern, enthält.

10. Zusammensetzung nach einem der Ansprüche 1 bis 9, **dadurch gekennzeichnet, dass** diese in Form einer Emulsion (Öl/Wasser und Wasser/Öl), als Milch, als Lotion, als Salbe, in gelierenden und viskosen spannungsaktiven und emulgierenden Polymeren, als Pommade, als Shampoo, als Seife, als Gel, als Puder, als Stick oder Stift, als Spray, als Körperöl, als Gesichtsmaske oder als Pflaster, vorliegt.

11. Nicht-therapeutische Verwendung einer Zusammensetzung nach einem der Ansprüche 1 bis 10 als kosmetischer Wirkstoff zur Stimulierung der Synthese von Laminin V, der Synthese von Collagen IV, der Synthese von Collagen VII, der Synthese von Collagen XVII und/oder der Synthese von Integrin β4.

12. Verwendung nach Anspruch 11 zur Verbesserung der durch die endogene oder exogene Degeneration der Basalmembran verursachten schlaffen, rauen und faltigen Haut.

13. Verwendung nach einem der Ansprüche 11 und 12 zur Verhinderung der Bildung von schlaffer, rauer und faltiger Haut.

14. Verwendung nach einem der Ansprüche 11 bis 13 in Kombination mit mindestens einem zusätzlichen Hautpflegewirkstoff.

15. Verbindungen der in Anspruch 1 definierten allgemeinen Formel (I), worin X $-NR^2-$ bedeutet und sowohl $R^1$ als auch $R^2$ von H verschieden sind.

16. Verbindungen entsprechend der in Anspruch 1 definierten allgemeinen Formel (I), worin aber $XR^1$ zusammen mit X in der Bedeutungsmöglichkeit -NH den Rest einer alpha-Aminosäure bedeutet.

## Claims

1. Composition, in particular a composition which can be applied topically, comprising at least one compound of the general formula (I),

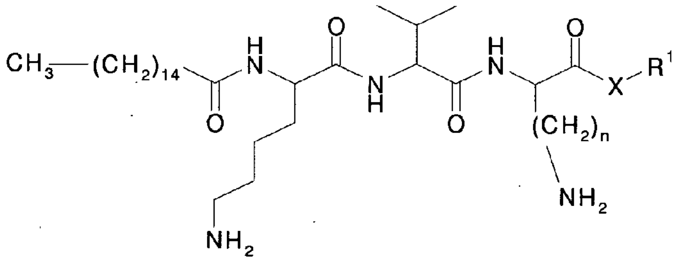

(I)

in which
$R^1$ is H, $C_1$-$C_{20}$-alkyl, cycloalkyl with no more than 8 C, or aryl-$C_1$-$C_4$-alkyl with no more than 10 C in the aryl moiety,
n is 1 - 4,
X is -O-, -NH-, or $NR^2$-, and
$R^2$ is H or $C_1$-$C_{20}$-alkyl;
and at least one compound corresponding to the above formula (I), but in which
$XR^1$ with X having the possible meaning of -NH- is the residue of an alpha-amino acid.

2. The composition according to claim 1, **characterized in that** the compounds defined in claim 1 are present in the form of dermatologically acceptable salts.

3. The composition according to claim 1 or claim 2, **characterized in that** in the compounds defined in claim 1, the amino acid residues are present as racemate or in their enantiomerically pure form L and D.

4. The composition according to any one of the claims 1 to 3, **characterized in that** a compound of the formula (I) defined in claim 1 in which $R^1$ is H or $C_1$-$C_{20}$-alkyl, n is 2 or 4, and X is -O-, is combined with compounds corresponding to the general formula (I) defined in claim 1, but in which $XR^1$ with X having the possible meaning of -NH- is the residue of a natural alpha-amino acid, and n is 2.

5. The composition according to claim 4, **characterized in that** the combination partners are Palm-Lys-Val-Dab-OH and Palm-Lys-Val-Dab-Thr-OH.

6. The composition according to any one of the claims 1 to 5, **characterized in that** the same contains the compounds mentioned in these claims in a concentration in the range of 0.5 to 5000 ppm (w/w), preferably in the range of 1 to 1000 ppm (w/w).

7. A composition, in particular a composition which can be applied topically, according to any one of the claims 1 to 6, **characterized in that** it contains in addition a safe and effective amount of at least one further skin care active ingredient selected from the group consisting of active ingredients for desquamation, anti-acne agents, vitamin B3 compounds, retinoids, di-, tri-, tetra-, penta- and hexapeptides and derivates thereof, hydroxy acids, radical scavengers, anti-inflammatory agents, skin tanning agents, skin lightening agents, anti-cellulite agents, flavonoids, antimicrobial agents, skin-healing agents, antimycotic agents, sunscreen agents, farnesol, phytantriol, allantoin, glucosamine and mixtures thereof.

8. The composition according to any one of the claims 1 to 7, **characterized in that** it contains in addition a dermatological carrier.

9. The composition according to any one of the claims 1 to 8, **characterized in that** the same contains the active compounds in solution, as dispersion, as emulsion or encapsulated in carriers, preferably as macro-, micro- or nanocapsules, in liposomes or chylomicrons, or enclosed in macro-, micro- or nano-particles or in microsponges, or absorbed on powdered organic polymers, talc, bentonite and related mineral carriers.

**10.** The composition according to any one of the claims 1 to 9, **characterized in that** the same is present in the form of an emulsion (oil/water and water/oil), as milk, as lotion, as ointment, in gelling and viscous, tensioactive and emulsifying polymers, as pomade, as shampoo, as soap, as gel, as powder, as stick or bar, as spray, as body oil, as face mask or as plaster.

**11.** A non-therapeutic use of a composition according to any one of the claims 1 to 10 as cosmetic active ingredient for stimulating the synthesis of laminin V, the synthesis of collagen IV, the synthesis of collagen VII, the synthesis of collagen XVII, and/or the synthesis of integrin β4.

**12.** The use according to claim 11 for improving lax, rough and wrinkled skin caused by endogenous and exogenous degeneration of the basal membrane.

**13.** The use according to any one of the claims 11 and 12 for preventing the formation of lax, rough and wrinkled skin.

**14.** The use according to any one of the claims 11 to 13 in combination with at least one additional skin care agent.

**15.** Compounds of the general formula (I) defined in claim 1, in which X is $-NR^2-$ and $R^1$ as well as $R^2$ are different from H.

**16.** Compounds of the general formula (I) defined in claim 1, but in which $XR^1$ together with X having the possible meaning of -NH- is the residue of an alpha-amino acid.

## Revendications

**1.** Composition, en particulier une composition applicable par voie topique, contenant au moins un composé de la formule générale (I) :

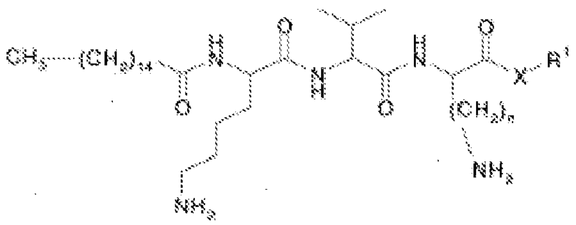

(I)

dans laquelle :

- $R^1$ représente H, alkyle en $C_1$-$C_{20}$, cycloalkyle avec au plus 8 C ou aryl-alkyle en $C_1$-$C_4$ avec au plus 10 C dans la fraction aryle ;
- n vaut 1-4 ;
- X représente -O-, -NH- ou $-NR^2-$ et
- $R^2$ représente H ou alkyle en $C_1$-$C_{20}$ ;

ainsi qu'au moins un composé correspondant à la formule (I) ci-dessus, mais dans laquelle :

- $XR^1$, avec X représentant -NH-, représente le reste d'un alpha-aminoacide.

**2.** Composition selon la revendication 1, **caractérisée par le fait que** les composés définis dans la revendication 1 se présentent sous la forme de sels dermatologiquement compatibles.

**3.** Composition selon l'une des revendications 1 ou 2, **caractérisée par le fait que**, dans les composés définis dans la revendication 1, les restes d'aminoacide se présentent sous la forme de racémates ou dans leur forme L et D énantiomériquement pure.

**4.** Composition selon l'une des revendications 1 à 3, **caractérisée par le fait qu'**un composé de la formule (I) définie dans la revendication 1, dans laquelle $R^1$ représente H ou alkyle en $C_1$-$C_{20}$, n vaut 2 ou 4 et X représente -O-, est

combiné avec un composé correspondant à la formule générale (I) définie dans la revendication 1, mais dans laquelle $XR^1$, avec X représentant -NH-, représente le reste d'un alpha-aminoacide naturel, et n vaut 2.

5. Composition selon la revendication 4, **caractérisée par le fait que** les partenaires de combinaison sont Palm-Lys-Val-Dab-OH et Palm-Lys-Val-Dab-Thr-OH.

6. Composition selon l'une quelconque des revendications 1 à 5, **caractérisée par le fait qu'**elle contient les composés mentionnés dans ces revendications dans une concentration se situant dans la plage de 0,5 à 5000 ppm (p/p), de préférence dans la plage de 1 à 1000 ppm (P/P).

7. Composition, en particulier une composition applicable par voie topique, selon l'une des revendications 1 à 6, **caractérisée par le fait qu'**elle contient en outre une quantité sans danger et efficace d'au moins un autre agent de soin dermatologique, choisi dans le groupe constitué par les agents pour la desquamation, les agents anti-acné, les composés de la vitamine B3, les rétinoïdes, les di-, tri-, tétra-, penta- et hexapeptides et leurs dérivés, les acides hydroxylés, les désactivateurs de radicaux, les agents anti-inflammatoires, les agents de bronzage de la peau, les agents d'éclaircissement de la peau, les agents anti-cellulite, les flavonoïdes, les agents antimicrobiens, les agents de cicatrisation de la peau, les agents antimicotiques, les agents de protection solaire, le farnésol, le phytantriol, l'allantoïne, la glucosamine et leurs mélanges.

8. Composition selon l'une des revendications 1 à 7, **caractérisée par le fait qu'**elle contient en outre un support dermatologique.

9. Composition selon l'une des revendications 1 à 8, **caractérisée par le fait qu'**elle contient les composés actifs en solution, comme dispersion, comme émulsion ou encapsulés dans des supports, de préférence sous forme de macro-, micro- ou nanocapsules, dans des liposomes ou des chylomicrons, ou enfermés dans des macro-, micro- ou nanoparticules ou dans des microéponges ou absorbés sur des polymères organiques pulvérulents, du talc, de la bentonite et des supports minéraux apparentés.

10. Composition selon l'une des revendications 1 à 9, **caractérisée par le fait qu'**elle se présente sous la forme d'une émulsion (huile/eau et eau/huile), comme lait, comme lotion, comme crème, dans des polymères tensio-actifs et émulsifiants gélifiants et visqueux, comme pommade, comme shampoing, comme savon, comme gel, comme poudre, comme bâtonnet ou crayon, comme pulvérisation, comme huile pour le corps, comme masque pour le visage ou comme patch.

11. Utilisation non thérapeutique d'une composition telle que définie à l'une des revendications 1 à 10 comme agent cosmétique pour stimuler la synthèse de la laminine V, la synthèse du collagène IV, la synthèse du collagène VII, la synthèse du collagène XVII et/ou la synthèse de l'intégrine β4.

12. Utilisation selon la revendication 11, pour améliorer la peau molle, rugueuse et ridée provoquée par la dégénérescence endogène ou exogène de la membrane basale.

13. Utilisation selon l'une des revendications 11 et 12 pour empêcher la formation de peau molle, rugueuse et ridée.

14. Utilisation selon l'une des revendications 11 à 13 en combinaison avec au moins un agent de soin dermatologique supplémentaire.

15. Composés de la formule générale (I) dans la revendication 1, dans laquelle X représente $-NR^2-$ et non seulement $R^1$ mais encore $R^2$ sont différents de H.

16. Composés correspondant à la formule générale (I) définie dans la revendication 1, mais dans laquelle $XR^1$, avec X représentant -NH-, représente le reste d'un alpha-aminoacide.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2004099237 A
- US 08834010 B
- WO 9739733 A1
- WO 03037933 A
- EP 0674274501521 A
- EP 2006003998 W


### In der Beschreibung aufgeführte Nicht-Patentliteratur


- **M. Bodanszky.** The Practice of Peptide Synthesis. Springer Verlag, 1994